# EUROPÄISCHE PATENTANMELDUNG

(11) **EP 0 558 451 A1**
(43) Veröffentlichungstag der Anmeldung: **01.09.1993**
(21) Anmeldenummer: 93810104.5
(22) Anmeldetag: 19.02.1993
(51) Int. Cl.: C08J 3/24, C08G 59/50

(54) **Verwendung von gehärtetem Material auf Basis von Epoxidharzen und Polyaminen zur Herstellung von orthopädischen Hilfsmitteln**

(30) Priorität: 28.02.1992 CH 621/92
(71) Anmelder: CIBA-GEIGY AG, CH-4002 Basel (CH)
(72) Erfinder: Urban, Klaus D., Dr., A-1010 Wien (AT); Felzl, Hans R., A-1190 Wien (AT)

(57) **Zusammenfassung**

Die vorliegende Erfindung betrifft ein Verfahren zur Herstellung von orthopädischen Hilfsmitteln aus einem Halbzeug, das aus gehärtetem Material auf Basis eines oder mehrerer Epoxidharze und eines oder mehrerer Polyamine als wesentlichen Polymerkomponenten erhältlich ist, bei dem das gehärtete Material nach Erwärmung über den Glasübergangspunkt umgeformt und wieder abgekühlt wird, die Verwendung des genannten Materials zur Herstellung formangepasster orthopädischer Hilfsmittel und die entsprechenden orthopädischen Hilfsmittel.

## Beschreibung

Die vorliegende Erfindung betrifft ein Verfahren zur Herstellung von orthopädischen Hilfsmitteln, die Verwendung von gehärtetem Material, das aus Zusammensetzungen auf Basis von Epoxidharzen und Polyaminen als wesentlichen Polymerkomponenten erhältlich ist, zur Herstellung orthopädischer Hilfsmittel sowie entsprechende orthopädische Hilfsmittel.

Orthopädische Hilfsmittel, wie Schuheinlagen oder Korsette, beispielsweise zur Rückgratkorrektur bei Haltungsschäden, müssen im allgemeinen in eine ganz bestimmte, individuell auf einen bestimmten Träger abgestimmte Form gebracht werden.

Unter anderem, um diese Formanpassung in einfacher Weise zu ermöglichen, werden derartige orthopädische Hilfsmittel seit einiger Zeit vermehrt aus Polymethylmethacrylat (PMMA), also einem thermoplastischen Kunststoff, hergestellt. Hierbei wird im allgemeinen ein Halbzeug aus dem Material, z. B. eine Polymethacrylatplatte, zunächst mechanisch vorgeformt, wobei übliche mechanische Formgebungsverfahren zur Anwendung kommen, wie beispielsweise Schneiden, Fräsen, Bohren, Schleifen oder Polieren. Die Endformung, d. h. die genaue Anpassung des hierbei erhaltenen Rohlings an die Erfordernisse bei einem bestimmten Patienten erfolgt schliesslich durch Warmformen, also durch eine Umformung, bei der das Material soweit erwärmt wird, dass es elastisch ist.

Polymethylmethacrylat weist jedoch einige Nachteile bei dem oben geschilderten Prozess auf, die ein Ersatzmaterial wünschenswert machen. So beginnt PMMA bereits bei relativ niedrigen Temperaturen zu erweichen und kann daher nur schlecht mechanisch bearbeitet werden, da es hierbei z. B. leicht zu einem Verkleben der Werkzeuge kommt oder das Material Fäden zieht. Andererseits sind für die thermische Endformung wiederum relativ hohe Temperaturen, um etwa 180 °C, erforderlich, da nur nach einer Verformung bei solch hohen Temperaturen ein ausreichend geringes Rückstellbestreben von PMMA gewährleistet ist. Abgesehen vom hierfür nötigen Aufwand, besteht bei Erwärmung von Plexiglasmaterial auf Temperaturen dieser Höhe immer die Gefahr von Blausäureemissionen.

Die vorliegende Erfindung hat die Aufgabe, ein Material für die Verwendung als Halbzeug zur Herstellung formangepasster orthopädischer Hilfsmittel anzugeben, das die Nachteile von PMMA nicht aufweist, sowie ein Verfahren zur Herstellung der genannten orthopädischen Hilfsmittel.

Es hat sich nun gezeigt, dass gehärtetes Material, das aus einer Zusammensetzung auf Basis eines oder mehrerer Epoxidharze und eines oder mehrerer Polyamine als wesentlichen Polymerkomponenten erhältlich ist, ein geeignetes Material für orthopädischen Hilfsmittel, wie den oben erwähnten, ist. Besonders überraschend ist hierbei, dass sich amingehärtetes Epoxidharzmaterial, das im Gegensatz zum Thermoplasten PMMA ja einen Duroplasten darstellt, thermisch so grossflächig verformen lässt, wie dies bei der Herstellung orthopädischer Hilfsmittel in aller Regel erforderlich ist.

Die vorliegende Erfindung betrifft daher die Verwendung von gehärtetem Material, das aus einer Zusammensetzung auf Basis eines oder mehrerer Epoxidharze und eines oder mehrerer Polyamine als wesentlichen Polymerkomponenten erhältlich ist, zur Herstellung formangepasster orthopädischer Hilfsmittel.

Sie betrifft weiterhin ein Verfahren zur Herstellung von orthopädischen Hilfsmitteln aus einem Halbzeug, das aus gehärtetem Material auf Basis eines oder mehrerer Epoxidharze und eines oder mehrerer Polyamine als wesentlichen Polymerkomponenten erhältlich ist, bei dem das gehärtete Material nach Erwärmung über den Glasübergangspunkt umgeformt und wieder abgekühlt wird.

Die Epoxidharze zur Herstellung des gehärteten Materials sind vorzugsweise aus cycloaliphatischen Epoxidharzen, Epoxidharzen auf Basis von 2,2-Bis-(4-hydroxyphenyl)propan (Bisphenol A), Bis-(4-hydroxyphenyl)methan (Bisphenol F) oder von Novolaken, also unter sauren Bedingungen erhaltenen Kondensationsprodukten von Phenolen und Formaldehyd, ausgewählt. Epoxidharze der genannten Art sind allgemein bekannt und beispielsweise in LEE & NEVILLE "Handbook of Epoxy Resins", McGraw-Hill, Inc., New-York, 1967, Kapitel 2, beschrieben. Solche Epoxidharze sind auch in vielfältiger Form im Handel erhältlich.

Besonders bevorzugt sind Di- oder Polyglycidylether bzw. Di- oder Poly-(β-methylglycidyl)ether von Bisphenol A und von Phenol- sowie Kresol-Novolaken. Diese können auch sehr gut im Gemisch angewandt werden.

Bevorzugt ist weiterhin die Verwendung von solchem Material, bei dem die Polyamine aus aromatischen Polyaminen, cycloaliphatischen Polyaminen und aliphatischen sowie heteroaliphatischen Polyaminen ausgewählt sind. Unter "heteroaliphabschen Aminen" werden hier Amine verstanden, die Heteroatome in der aliphatischen Kette enthalten, bei spielsweise Polyetherpolyamine. Besonders geeignet sind hierbei Polyamine, die primäre Aminogruppen aufweisen. Geeignete Polyamine der genannten Typen sind ebenfalls allgemein bekannt. Ein Überblick über Amine dieser Art wird in LEE & NEVILLE "Handbook of Epoxy Resins", McGraw-Hill, Inc., New-York, 1967, Kapitel 7 und 8 gegeben. Viele für die Erfindung geeignete Polyamine des oben genannten Typs sind auch kommerziell erhältlich.

Zur Herstellung der erfindungsgemäss verwendeten härtbaren Massen werden Epoxidharze und Amine in üblichen Mischungsverhältnissen eingesetzt, d. h. im allgemeinen in annähernd äquivalenten Mengen, z. B. in Mengen von 0,8 bis 1,5 und insbesondere von 1 bis 1,3 Äquivalenten Aminowasserstoffatom auf 1 Epoxidäquivalent.

Es hat sich besonders bewährt, wenn das erfindungsgemäss verwendete Material flexibilisierende Bestandteile enthält. Die Flexibilisierung kann beispielsweise durch Verwendung von Epoxidharzen und/oder Polyaminen erreicht werden, die dadurch modifiziert sind, dass nicht starre Molekülgruppen zwischen den zur Vernetzung verwendeten funktionellen Gruppen eingebaut sind, z. B. längere Ketten von Atomen oder Atomgruppen, die überwiegend mit Hilfe von Einzelbindungen verknüpft sind. Die Kettenlänge der flexibilisierenden Gruppen sollte hierbei bevorzugt bei 8 und mehr Atomen oder Atomgruppen liegen. Zusätzlich können monofunktionelle Verbindungen eingesetzt werden, die eine entsprechende flexibilisierende Molekülgruppe enthalten, beispielsweise entsprechend aufgebaute Monoamine.

Bei einer besonders bevorzugten Ausführungsform der Erfindung wird Material verwendet, bei dem die Polyamine aus cycloaliphatischen Polyaminen sowie aliphatischen und heteroaliphatischen Polyaminen ausgewählt sind, wobei die aliphatischen und heteroaliphatischen Polyamine mindestens eine Aminogruppe aufweisen, die einen Abstand von 8 oder mehr Atomen zur nächsten Aminogruppe hat.

Als cycloaliphatische Polyamine kommen insbesondere Derivate des Cyclohexans zur Anwendung, z. B. Diaminocyclohexan oder Bis-(4-aminocyclohexyl)methan und ähnliche, die gegebenenfalls auch substituiert sein, insbesondere C₁-C₄-Alkyl-Substituenten aufweisen können, so wie z. B. Bis-(3-methyl-4-amino-cyclohexyl)methan. Ebenfalls gut geeignet sind Amine der allgemeinen Formel R-NH-(CH₂)ₓ-(CHR¹)_{y}-(CH₂)_{z}-NH₂, worin R eine Cycloalkylgruppe, insbesondere von 1 bis 10 Kohlenstoffatomen, oder eine Cycloalkylalkylgruppe, insbesondere von 1 bis 20 Kohlenstoffatomen bedeutet und beide Typen von Gruppen gegebenenfalls auch einen oder mehrere Halogen-, C₁-C₁₀-Alkyl- oder Arylsubstituenten aufweisen können, und R¹ eine C₁-C₁₀-Alkylgruppe bedeutet, die Indices x und z unabhängig voneinander einen ganzzahligen Wert von 0 bis 10 und y einen ganzzahligen Wert von 0 oder 1 annehmen können, jedoch x, y und z niemals alle gleichzeitig 0 sein können. Ein Beispiel für eine Verbindung dieses Typs ist 1-(N-Hexahydrobenzylamino)-3-aminopropan. Aminoverbindungen, wie sie in diesem Abschnitt genannt sind, sind bereits für Epoxidmassen zur Herstellung von Brillenrahmen zur Anwendung gekommen und sind z. B. im britischen Patent Nr. 1,169,121 beschrieben. Als cycloaliphatisches Polyamin ganz besonders bevorzugt ist 3-(Aminomethyl)-3,5,5-trimethylcyclohexylamin (Isophorondiamin).

Beispiele für aliphatische oder heteroaliphatische Polyamine mit mindestens einer Aminogruppe, die einen Abstand von 8 oder mehr Atomen zur nächsten Aminogruppe aufweist, sind 1,8-Diaminooctan, 1,10-Diaminodecan, oder 1,12-Diaminododecan. Solche und ähnliche Diamine können leicht aus den entsprechenden Dinitrilen erhalten werden, die ihrerseits z. B. durch Entwässerung von entsprechenden Säureamiden hergestellt werden können. Die Dinitrile werden dazu unter Druck im Autoklaven mit flüssigem Ammoniak (4 - 8 Mol pro Mol Dinitril) und/oder Methanol als Lösungsmittel und Raney-Nickel als Katalysator bei Temperaturen bis ca. 125 °C hydriert. Weitere Beispiele für Polyamine dieses Typs sind Polyoxyalkylenpolyamine, z. B. Polyoxypropylenpolyamine, wie sie beispielsweise unter der Bezeichnung Jeffamine® im Handel sind. Ein besonders bevorzugtes Polyetherpolyamin ist Dioxadodecandiamin, eine bekannte Verbindung, die ebenfalls im Handel erhältlich ist.

Es kann auch zweckmässig sein, primäre Polyamine zunächst mit einem Epoxidharz zu linearen Addukten vorzureagieren und in dieser Form als Härter bei der Herstellung des erfindungsgemäss verwendeten Materials einzusetzen. Die Herstellung eines solchen Adduktes erfolgt bevorzugt bei erhöhter Temperatur, z. B. bei 80 bis 200 °C.

Als besonders günstig hat sich der erfindungsgemässe Einsatz von solchem Material erwiesen, das aus einem oder mehreren Epoxidharzen und einem Gemisch von cycloaliphatischen Polyaminen und aliphatischen oder heteroaliphabschen Polyaminen als wesentlichen Polymerkomponenten erhältlich ist, wobei die aliphatischen und heteroaliphatischen Polyamine mindestens eine Aminogruppe aufweisen, die einen Abstand von 8 oder mehr Atomen zur nächsten Aminogruppe hat. Die aliphatischen bzw. heteroaliphatischen Polyamine werden hierbei bevorzugt in einem Gewichtsverhältnis von 1:1 bis 10:1 zueinander eingesetzt. Besonders günstig ist es, wenn das Polyamingemisch aus Isophorondiamin und Dioxadodecandiamin besteht. Das Mischungsverhältnis von Dioxadodecandiamin zu Isophorondiamin beträgt hierbei günstigerweise 1 : 1 bis 5 : 1, ebenfalls bezogen auf die Gewichte, insbesondere 2 : 1 bis 4 : 1.

In vielen Fällen enthalten die Zusammensetzungen, aus denen die erfindungsgemäss verwendeten Materialien erhältlich sind, zweckmässig noch weitere übliche Zusatzstoffe neben den Epoxidharzen und Polyaminen, insbesondere Pigmente, Farbstoffe, Verarbeitungshilfsmittel, Beschleuniger sowie Füll- und Verstärkungsstoffe usw..

Zur Herstellung des amingehärteten Epoxidharzmaterials sind die üblichen in der Epoxidtechnologie bekannten Verfahren geeignet, z. B. freier Guss, Spritzguss, Laminieren, Sheet Molding oder Druckgelieren (ADG). Bevorzugt wird das Material danach noch einige Zeit, z. B. für einen Zeitraum von 30 Minuten bis zu mehreren Stunden bei 100 bis 150 °C getempert. Das Material kann in dieser Form gelagert und vertrieben werden und bei Bedarf als Halbzeug zur Herstellung beliebiger orthopädischer Hilfsmittel eingesetzt werden. Das Halbzeug hat zweckmässigerweise die Form von Platten, beispielsweise einer Stärke von 2 bis 10 Millimetern. Selbstverständlich kann es in manchen Fällen aber auch sinnvoll sein, bereits das Halbzeug in einer Weise vorgeformt herzustellen, die für eine bestimmte Formgebung vorangepasst ist.

Bei der Herstellung der orthopädischen Hilfsmittel aus den genannten Halbzeugen erfolgt noch eine Formanpassung durch Warmformen, bei der im allgemeinen die endgültig angestrebte Form des orthopädischen Hilfsmittels erzeugt wird. Hierzu wird das gehärtete Material zunächst auf eine Temperatur über dem Glasübergangspunkt erwärmt. Im allgemeinen werden hierbei Temperaturen im Bereich von 80 bis 150 °C angewandt, insbesondere Temperaturen von 100 bis 120 °C, also wesentlich niedrigere Temperaturen, als sie bei der Warmformung von PMMA-Material erforderlich sind. Als Wärmequellen können z. B. Wärmeschränke, Infrarotstrahler, Heissluft, Gasflammen oder heisse Flüssigkeiten, wie Öle, geschmolzenen Fette oder in geeigneten Fällen auch Wasser angewandt werden. Danach wird das Material in beliebiger Weise, beispielsweise von freier Hand oder mit Hilfe einer Schablone oder eines anderen speziellen Formwerkzeuges, z. B. einer Presse, verformt und in der gewünschten Form fixiert wieder auf eine Temperatur unter dem Glasübergangspunkt des Materials abgekühlt. Die Abkühlung kann gewünschtenfalls z. B. mit Hilfe kalter Pressluft oder kalten Wassers beschleunigt werden. Beim Abkühlen unter die Glasübergangstemperatur wird das gehärtete und umgeformte Material günstigerweise in der gewählten Form fixiert.

Zusätzlich zu der geschilderten thermischen Verformung können auch noch mechanische Formgebungsverfahren erforderlich oder zweckmässig sein, z. B. Schneiden, Fräsen, Bohren, Polieren oder auch andere. Im Gegensatz zu Thermoplasten, insbesondere zu PMMA, besteht bei dem erfindungsgemäss verwendeten gehärteten Epoxidmaterial keine Gefahr einer vorzeitigen Erweichnung mit den eingangs erwähnten negativen Folgen.

Schliesslich betrifft die Erfindung noch die einer vorgegebenen Form zumindest teilweise durch Warmformung angepassten orthopädischen Hilfsmittel, die unter Verwendung von gehärtetem Material der oben beschriebenen Art erhalten wurden.

Beispiel: 100 Gramm eines Epoxidharzes auf Basis von Bisphenol A mit einem Molekulargewicht unter 700 und einem Epoxidäquivalent von 180 - 190 g/Eq (ARALDIT®GY 260) werden mit 56 Gramm eines Härters vermischt, der ein Addukt aus 36 Gewichtsteilen des gleichen Harzes, 47 Gewichtsteilen Dioxadodecandiamin und 17 Gewichtsteilen Isophorondiamin (VESTAMIN®IPD) darstellt. Das Gemisch wird bei Raumtemperatur und einem Druck von ca. 1330 Pa zwei Stunden lang entgast. Durch eine fünfminütige Druckgelierung bei 120 °C wird das entgaste Gemisch zu ca. 5 Millimeter starken Platten verarbeitet. Die entformten Platten werden danach noch eine Stunde bei 120 °C getempert.

Aus diesem Halbzeug werden Rohlinge geschnitten und in einer Presse bei etwa 120 °C und bis zu ca. 4 bar Überdruck in eine dreidimensionale Form gebracht. Nach Abkühlung in der Presse auf Raumtemperatur wird das Material entformt, und behält das Material diese Form bei.

## Patentansprüche

1. Verfahren zur Herstellung von orthopädischen Hilfsmitteln aus einem Halbzeug, das aus gehärtetem Material auf Basis eines oder mehrerer Epoxidharze und eines oder mehrerer Polyamine als wesentlichen Polymerkomponenten erhältlich ist, bei dem das gehärtete Material nach Erwärmung über den Glasübergangspunkt umgeformt und wieder abgekühlt wird.

2. Verfahren gemäss Anspruch 1, wobei die Epoxidharze aus cycloaliphatischen Epoxidharzen, Epoxidharzen auf Basis von Bisphenol A, Epoxidharzen auf Basis von Bisphenol F und Epoxidharzen auf Basis von Novolaken ausgewählt sind.

3. Verfahren gemäss Anspruch 1 oder 2, wobei die Polyamine aus aromatischen Polyaminen, cycloaliphatischen Polyaminen, aliphatischen Polyaminen und heteroaliphatischen Polyaminen ausgewählt sind.

4. Verfahren gemäss Anspruch 3, wobei die Polyamine aus cycloaliphatischen Polyaminen, aliphatischen Polyaminen und heteroaliphatischen Polyaminen ausgewählt sind, wobei die aliphatischen und heteroaliphatischen Polyamine mindestens eine Aminogruppe aufweisen, die einen Abstand von 8 oder mehr Atomen zur nächsten Aminogruppe hat.

5. Verfahren gemäss Anspruch 4, wobei das gehärtete Kunstoffmaterial aus einem oder mehreren Epoxidharzen und einem Gemisch von Polyaminen, ausgewählt aus cycloaliphatischen Polyaminen, aliphatischen Polyaminen und heteroaliphatischen Polyaminen, erhältlich ist, wobei die aliphatischen und heteroaliphatischen Polyamine mindestens eine Aminogruppe aufweisen, die einen Abstand von 8 oder mehr Atomen zur nächsten Aminogruppe hat.

6. Verfahren gemäss Anspruch 5, wobei das Polyamingemisch aus Isophorondiamin und Dioxadodecandiamin besteht.

7. Verfahren gemäss mindestens einem der Ansprüche 1 bis 6, wobei das gehärtete Material aus einer Zusammensetzung erhältlich ist, die neben den Epoxidharzen und Polyaminen weitere Zusatzstoffe enthält, insbesondere Pigmente, Farbstoffe, Verarbeitungshilfsmittel, Beschleuniger sowie Füll- und Verstärkungsstoffe.

8. Verfahren gemäss mindestens einem der Ansprüche 1 bis 6, wobei das Halbzeug zusätzlich einer mechanischen Formgebung unterzogen wird.

9. Verwendung von gehärtetem Material, das aus einer Zusammensetzung auf Basis eines oder mehrerer Epoxidharze und eines oder mehrerer Polyamine als wesentlichen Polymerkomponenten erhältlich ist, zur Herstellung formangepasster orthopädischer Hilfsmittel.

10. Einer vorgegebenen Form zumindest teilweise durch Warmformung angepasstes orthopädisches Hilfsmittel erhalten unter Verwendung von gehärtetem Material, das aus einer Zusammensetzung auf Basis eines oder mehrerer Epoxidharze und eines oder mehrerer Polyamine als wesentlichen Polymerkomponenten erhältlich ist.
